# EUROPEAN PATENT SPECIFICATION

(11) **EP 1 520 012 B1**
(45) Date of publication and mention of the grant of the patent: **07.01.2009**
(21) Application number: 03761447.6
(22) Date of filing: 01.07.2003
(51) Int. Cl.: C12N 9/00, C12N 1/38

(54) **MONOPROPYLENE GLYCOL ADDED TO FERMENTATION**
MONOPROPYLENGLYKOL ALS FERMENTATIONSZUSATZ
ADJONCTION DE MONOPROPYLENE GLYCOL A UNE FERMENTATION

(30) Priority: 01.07.2002 DK 200201021
(43) Date of publication of application: 06.04.2005
(73) Proprietor: Novozymes A/S, 2880 Bagsvaerd (DK)
(72) Inventor: KAASGAARD, Svend, DK-2860 Soborg (DK); BANKE, Niels, DK-2860 Soborg (DK); HANSEN, Kim, Uhre, DK-4400 Kalundborg (DK)
(74) Representative: Kofoed, Gertrud Sonne
(86) International application number: PCT/DK2003/000455
(87) International publication number: WO 2004/003187

(56) References cited:
- WO-A-03/014339
- CH-A- 667 673
- DD-A- 153 495
- GB-A- 1 001 173
- US-A- 4 016 039
- US-A- 4 673 647
- US-A- 5 260 202
- PATENT ABSTRACTS OF JAPAN vol. 009, no. 153 (C-288), 27 June 1985 (1985-06-27) & JP 60 030682 A (AMANO SEIYAKU KK), 16 February 1985 (1985-02-16)
- ESTEVE-ROMERO JOSEP S ET AL: "Purification of thermamylase in multicompartment electrolyzers with isoelectric membranes: The problem of protein solubility." ELECTROPHORESIS, vol. 17, no. 7, 1996, pages 1242-1247, XP001104576 ISSN: 0173-0835

## Description

### TECHNICAL FIELD

The present invention relates to a method of increasing solubility of an enzyme of interest during fermentation.

### BACKGROUND ART

Formation of polypeptide crystals/amorphous precipitate during fermentation is today seen frequently because the fermentation yields are getting higher and higher due to optimization of the fermentation recipes and/or due to identification/development or construction of more efficient production organisms.

In such cases, the polypeptides are fermented in yields that are above their solubility limit, meaning that they may be present in the culture broth in a partly precipitated form. The precipitate may be in the form of crystals or as amorphous precipitates.

This causes problems in recovery where special measures have to be taken to solubilize the crystals/amorphous precipitate before removing the cells and other solids from the culture broth. These measures often result in yield losses.

The purpose of this invention is therefore to provide a simple and efficient solution to the above described problem.

US 5,260,202 describes that the recoverable yield of human albumin in a fermentation is increased by addition of a stabilizing agent comprising a polyoxyalkylene compound.

### SUMMARY OF THE INVENTION

It has surprisingly been found that the enzyme of interest can be prevented from crystallizing or precipitating by adding a carbohydrate and/or a polyol and/or a derivative thereof and/or a polymer to the culture medium before and/or during fermentation, wherein the microorganism is not, or only to a low extent, able to metabolize said carbohydrate and/or said polyol and/or said derivative thereof; in particular the present invention deals with:
A method for fermenting a bacterium, producing an enzyme of interest, in a culture medium of at least 50 litres, comprising:
   adding one or more compounds selected from the group consisting of 1,2-propandiol, 1,3-propandiol, ethylene glycol, trehalose, xylitol, arabitol, dulcitol, mannitol, erythritol, cellobiose, sorbitol and a polyether having an average molecular weight less than 1000, to the culture medium during fermentation, wherein the compound is low metabolizable measured by (OD_{III}-OD_{II})/(OD_{I}-OD_{II}) < 25%, and added in an amount of 0.5-10 % (w/w) of the culture medium, wherein
   OD_{I} is the amount of biomass measured as optical density (OD) at 650 nm with glucose as the only carbohydrate source;
   OD_{II} is the amount of biomass measured as optical density (OD) at 650 nm without glucose; and
   OD_{III} is the amount of biomass measured as optical density (OD) at 650 nm without glucose, but with the same C-mole of the compound to be tested".

### DETAILED DISCLOSURE OF THE INVENTION

The present invention deals with a new and surprisingly effective way of preventing the enzyme of interest to crystallize or precipitate during the fermentation.

We have surprisingly found that if small amounts of, e.g., 5 % w/w of monopropylene glycol (MPG) is present during the fermentation, the formation of crystals or amorphous precipitate can be avoided, significantly delayed or significantly reduced. The MPG is only a very poor carbon source for most microorganisms or is very poorly metabolized by most microorganisms, or not metabolized at all, so it can be added before starting the fermentation and/or added during the fermentation without affecting the cell growth and productivity of the peptide of interest significantly.

By avoiding formation of polypeptide crystals/amorphous precipitate during fermentation, a much more simple recovery process can be used resulting in higher yields.

### Microorganisms

The microorganism (the microbial strain) according to the invention may be obtained from microorganisms of any genus.

In a preferred embodiment, the enzyme of interest may be obtained from a bacterial source.

For example, the enzyme of interest may be obtained from a gram positive bacterium such as a *Bacillus* strain, *e.g., Bacillus alkalophilus*, *Bacillus amyloliquefaciens*, *Bacillus brevis, Bacillus circulans*, *Bacillus coagulans*, *Bacillus lautus*, *Bacillus lentus*, *Bacillus licheniformis*, *Bacillus megaterium*, *Bacillus stearothermophilus*, *Bacillus subtilis,* or *Bacillus thuringiensis*; or a *Streptomyces* strain, *e.g., Streptomyces lividans* or *Streptomyces murinus*; or from a gram negative bacterium, *e.g*., *E. coli* or *Pseudomonas* sp.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

For purposes of the present invention, the term "obtained from" as used herein in connection with a given source shall mean that the polypeptide of interest is produced by the source or by a cell in which a gene from the source has been inserted.

### Modification of the microorganism of interest

The microorganism used according to the present invention may be modified in such a way that it is not, or only to a low extent, able to metabolize the chosen carbohydrate and/or polyol and/or derivative thereof; e.g., the original microorganism is able to metabolize glycerol or cyclodextrin but the modified microorganism is not, or only to a low extent.

### Polypeptide of interest

In a preferred embodiment, the protein is an enzyme, in particular a hydrolase (class EC 3 according to Enzyme Nomenclature; Recommendations of the Nomenclature Committee of the International Union of Biochemistry).

In a particular preferred embodiment the following hydrolases are preferred:
Proteases: Suitable proteases include those of animal, vegetable or microbial origin. Microbial origin is preferred. Chemically modified or protein engineered mutants are included. The protease may be an acid protease, a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include ALCALASE™, SAVINASE™, PRIMASE™, DURALASE™, ESPERASE™, RELASE™ and KANNASE™ (Novozymes A/S), MAXATASE™, MAXACAL™, MAXAPEM™, PROPERASE™, PURAFECT™, PURAFECT OXP™, FN2™, and FN3™ (Genencor International Inc.). Lipases: Suitable lipases include those of bacterial or fungal origin. Chemically modified or protein engineered mutants are included. Examples of useful lipases include lipases from *Humicola* (synonym *Thermomyces*), e.g. from *H. lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens*, *Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include LIPOLASE^{™}, LIPOLASE ULTRA^{™} and LIPEX™ (Novozymes A/S). Amylases: Suitable amylases (alpha and/or beta) include those of bacterial or fungal origin. Chemically modified or protein engineered mutants are included. Amylases include, for example, alpha-amylases obtained from *Bacillus,* e.g. a special strain of *B. licheniformis,* described in more detail in GB 1,296,839.

Examples of useful amylases are the variants described in WO 94/02597, WO 94/18314, WO 96/23873, WO 97/43424, and WO 01/66712, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are DURAMYL^{™}, TERMAMYL^{™}, FUNGAMYL^{™}, NATALASE™, TERMAMYL LC™, TERMAMYL SC™, LIQUIZYME-X™ and BAN^{™} (Novozymes A/S), RAPIDASE^{™} and PURASTAR^{™} (from Genencor International Inc.). Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US. 5,686,593, US 5,763,254, WO 95/24471, WO 98/12307 and PCT/DK98/00299.

Commercially available cellulases include CELLUZYME™, CAREZYME™ , and CAREZYME CORE™ (Novozymes A/S), CLAZINASE™, and PURADAX HA™ (Genencor International Inc.), and KAC-500(B)™ (Kao Corporation).

### Oxidoreductases

Oxidoreductases that may be treated according to the invention include peroxidases, and oxidases such as laccases, and catalases.

Other preferred hydrolases are carbohydrolases including MANNAWAY^{™}. Other preferred enzymes are transferases, lyases, isomerases, and ligases.

### Fermentations

The present invention may be useful for any fermentation in industrial scale, e.g. for any fermentation having culture media of at least 50 litres, preferably at least 100 litres, more preferably at least 500 litres, even more preferably at least 1000 litres, in particular at least 5000 litres.

The microbial strain may be fermented by any method known in the art. The fermentation medium may be a complex medium comprising complex nitrogen and/or carbon sources, such as soybean meal, soy protein, soy protein hydrolysate, cotton seed meal, corn steep liquor, yeast extract, casein, casein hydrolysate, potato protein, potato protein hydrolysate, molasses, and the like. The fermentation medium may be a chemically defined media, e.g. as defined in WO 98/37179.

The fermentation may be performed as a batch, a fed-batch, a repeated fed-batch or a continuous fermentation process.

In a fed-batch process, either none or part of the compounds comprising one or more of the structural and/or catalytic elements is added to the medium before the start of the fermentation and either all or the remaining part, respectively, of the compounds comprising one or more of the structural and/or catalytic elements is fed during the fermentation process. The compounds which are selected for feeding can be fed together or separate from each other to the fermentation process.

In a repeated fed-batch or a continuous fermentation process, the complete start medium is additionally fed during fermentation. The start medium can be fed together with or separate from the structural element feed(s). In a repeated fed-batch process, part of the fermentation broth comprising the biomass is removed at time intervals, whereas in a continuous process, the removal of part of the fermentation broth occurs continuously. The fermentation process is thereby replenished with a portion of fresh medium corresponding to the amount of withdrawn fermentation broth.

In a preferred embodiment of the invention, a fed-batch, a repeated fed-batch process or a continuous fermentation process is preferred.

### Carbohydrates

Slowly metabolizable carbohydrates such as pullulan, limit dextrin, and trehalose may be used according to the present invention.

In a particular embodiment of the invention the slowly metabolizable carbohydrate is added to the culture medium either prior to inoculation or after inoculation at an amount of at least 0.5% (w/w). The slowly metabolizable carbohydrate is added to the culture medium either prior to inoculation or after inoculation at an amount of up to 10% w/w; preferably at an amount of up to 8% w/w; more preferably at an amount of up to 6% w/w; more preferably at an amount of up to 5% w/w; more preferably at an amount of up to 4% w/w; more preferably at an amount of up to 3% w/w; more preferably at an amount of up to 2% w/w; even more preferably at an amount of up to 1% w/w.

### Polyols

A very useful subgroup of carbohydrates, polyols, may be used according to the invention. Any polyol may be used. However, a polyol selected from the group consisting of 1,2-propandiol (monopropylene glycol), 1,3-propandiol, glycerol, ethylene glycol, xylitol, arabitol, dulcitol, mannitol, erythritol, cellobiose and sorbitol, is preferred.

It is to be noted that some polyols, e.g. glycerol, are rather easily metabolized by most cells, but the uptake of e.g. glycerol can be blocked, meaning that glycerol may be used according to the present invention.

In a particular embodiment of the invention the polyol is added to the culture medium either prior to inoculation or after inoculation at an amount of at least 0.5% (w/w). The polyol is added to the culture medium either prior to inoculation or after inoculation at an amount of up to 10% w/w; preferably at an amount of up to 8% w/w; more preferably at an amount of up to 6% w/w; more preferably at an amount of up to 5% w/w; more preferably at an amount of up to 4% w/w; more preferably at an amount of up to 3% w/w; more preferably at an amount of up to 2% w/w; even more preferably at an amount of up to 1% w/w.

In some cases it may be an advantage to use a mixture of two or more polyols, e.g. glycerol and monopropylene glycol, or a mixture of a polyol and a slowly metabolizable carbohydrate.

### Derivatives

Another very useful subgroup of carbohydrates, derivatives, may be used according to the invention. Derivatives that may be used include maillard products, methyl glycosides, glucoronic acids, amino sugars, or N-acetyl glucosamines.

In a particular embodiment of the invention the derivative is added to the culture medium either prior to inoculation or after inoculation at an amount of at least 0.5% (w/w). The derivative is added to the culture medium either prior to inoculation or after inoculation at an amount of up to 10% w/w; preferably at an amount of up to 8% w/w; more preferably at an amount of up to 6% w/w; more preferably at an amount of up to 5% w/w; more preferably at an amount of up to 4% w/w; more preferably at an amount of up to 3% w/w; more preferably at an amount of up to 2% w/w; even more preferably at an amount of up to 1% w/w.

### Polymers

Polymers such as polyethers having an average molecular weight less than 1000; preferably an average molecular weight less than 900; more preferably an average molecular weight less than 800; even more preferably an average molecular weight less than 700, (e.g. polyethylene glycol 200 (PEG 200), polyethylene glycol 400 (PEG 400)), or their derivatives including block polymers or block copolymers of polyethylene oxide and polypropylene oxide, wherein the ends of the polymers may further be protected by an acyl group or an alkyl group, may also be used according to the present invention.

In a particular embodiment of the invention the polymer is added to the culture medium either prior to inoculation or after inoculation at an amount of at least 0.5% (w/w). The polymer is added to the culture medium either prior to inoculation or after inoculation at an amount of up to 10% w/w; preferably at an amount of up to 8% w/w; more preferably at an amount of up to 6% w/w; more preferably at an amount of up to 5% w/w; more preferably at an amount of up to 4% w/w; more preferably at an amount of up to 3% w/w; more preferably at an amount of up to 2% w/w; even more preferably at an amount of up to 1% w/w.

### Salts

In addition to adding a slowly metabolizable carbohydrate it may also be an advantage to add a salt to the fermentation medium (see e.g. Example 4).

A preferred salt is selected from the group consisting of a chloride, a sulphate, a phosphate, a nitrate, and an ammonium salt; e.g. NaCl, KCI, Na2SO4, K2SO4.

### Extent of metabolization

The following test may be used to check whether a microorganism, producing a polypeptide of interest, is not, or only to a low extent, able to metabolize a given compound:
A suitable media for the growth of the microorganism of interest is chosen.
   The media is characterized by the following parameters:
   a: The media contains glucose as the only carbohydrate source.
   b. When glucose is removed the media should only be able to support growth of a significantly lower biomass (less than 50%).

The growth of the microorganism of interest is then compared in the following 3 media:
I: Normal media (with glucose as the only carbohydrate source)
II: Media I without glucose
III: Media I without glucose, but with the same C-mol of the compound to be tested.

The growth is then followed for a period of 8 hr in the 3 above mentioned media. Inoculation is done with a concentration of biomass that will secure that the normal media is outgrown in 75% of the time frame. The amount of biomass is measured as optical density (OD) at 650 nm. OD obtained in the different media is measured.
The compound to be tested is defined as low metabolizable, if
(OD_{III}-OD_{II})/(OD_{I}-OD_{II}) < 25%; preferably
(OD_{III}-OD_{II})/(OD_{I}-OD_{II}) < 20%; more preferably
(OD_{III}-OD_{II})/(OD_{I}-OD_{II}) < 15%; more preferably
(OD_{III}-OD_{II})/(OD_{I}-OD_{II}) < 10%; more preferably
(OD_{III}-OD_{II})/(OD_{I}-OD_{II}) < 5%; more preferably
(OD_{III}-OD_{II})/(OD_{I}-OD_{II}) = 0%
In Example 1 various compounds are tested according to this test.

### Recovery of the polypeatide of interest

A further aspect of the invention concerns the downstream processing of the fermentation broth. After the fermentation process is ended, the polypeptide of interest may be recovered from the fermentation broth, using standard technology developed for the polypeptide of interest. The relevant downstream processing technology to be applied depends on the nature of the polypeptide of interest.

A process for the recovery of a polypeptide of interest from a fermentation broth will typically (but is not limited to) involve some or all of the following steps:
1) pre-treatment of broth (e.g. flocculation)
2) removal of cells and other solid material from broth (primary separation)
3) filtration
4) concentration
5) filtration
6) stabilization and standardization.

Apart from the unit operations listed above, a number of other recovery procedures and steps may be applied, e.g., pH-adjustments, variation in temperature, crystallization, treatment of the solution comprising the polypeptide of interest with active carbon, and use of various adsorbents.

By using the method of the invention the yield of the polypeptide of interest is much higher in the recovery when the crystal formation is reduced or eliminated by adding of , e.g. MPG, during fermentation.

The invention is further illustrated in the following examples, which are not intended to be in any way limiting to the scope of the invention as claimed.

### Example 1

### Evaluation of the suitability of different polyols and carbohydrates as carbon-sources for micro-organisms.

### Shake flask media:

### Med-F 18 shake flask medium (concentrations are after final mixing).

Part A: Bacto-peptone 0.5; Yeast Extract 0.5 g/I; Magnesium sulphate, 7H2O 0.5 g/l; Ammonium sulphate 2 g/I; Calcium chloride, 2H2O 0.1 g/I; Citric acid 50 mg/l; trace metals (MnSO4, H2O 2.5 mg/l; FeSO4, 7H2O 9.9 mg/l; CuSO4, 5H2O 1.0 mg/l; ZnCl2 1.0 mg/l); PLURONIC™ 0.1 g/I; pH adjusted to 6.7.
Part B: 5 g/I Potassiumdihydrogenphosphate pH adjusted to 6.7 with NaOH.
Part C: carbon source equivalent to 0.08 mol carbon per liter (e.g. 2.5 g/I glucose) Demineralized water is used for the preparation of all media.

After sterilization for 20 minutes at 121°C part A, B and C are mixed.

### Strain: Bacillus licheniformis

Procedure for shake flask evaluation of suitability of different polyols and carbohydrates as alternative carbon-sources:
First the cells were grown in a pre-culture that secured good growing cells.
Each shake flask is then inoculated with the same amount of cells based on the OD (650 nm) measurement.
An inoculum strength of OD x ml cell suspension = 80 was used in this case. Resulting in an OD=0.8 in the shake flask at time 0.
Three shake flasks of each type were inoculated.

Shake flask types:
I: Med-F 18 with the normal part C added, where C is glucose (resulting in a medium with 2.5 g/l of glucose (equivalent to 0.08 C-mol per Liter).
II: Med-F 18 without part C, resulting in a medium without any glucose
III: Med-F 18 with part C replaced by containing one of the following:
   - 2.1 g/l MPG (equivalent to 0.08 C-mol per Liter)
   - 2.1 g/l PEG 200 (equivalent to 0.08 C-mol per Liter) or
   - 2.4 g/I Sucrose (equivalent to 0.08 C-mol per Liter)
Resulting in media with 2.1 g/l MPG; 2.1 g/l PEG 200 or 2.4 g/I Sucrose, respectively.

By an error the media was prepared with 2.5 g/I MPG; 2.5 g/I PEG 200; and 2.5 g/I sucrose, respectively. This should therefore result in a lightly higher OD in these media compared to the intended media, if the compounds were easily metabolized.

The shake flasks were then incubated at 37°C at 300 rpm in 8 hr.
The OD (650 nm) was measured at 6 and 8 hr. and the following results were obtained:

**Table 1. Test of MPG: Average OD (650 nm) found for the three shake flask at time 6 and 8 hr.**

| OD (650 nm) Time [hr.] | OD_{I} | DO_{II} | OD_{III} | (OD_{III}-OD_{II}) (OD_{I}-OD_{II}) |
|---|---|---|---|---|
| 6 | 4.23 | 1.51 | 1.61 | 3.8% |
| 8 | 4.08 | 1.46 | 1.57 | 4.3% |

From the results it is clear that MPG is only very slowly or not at all metabolized by the strain used in this example. It is also clear that the culture is fully outgrown after 6 hr as the OD is not increased in medium I going from 6 to 8 hr.

**Table 2. Test of PEG 200: Average OD (650 nm) found for the three shake flask at time 6 and 8 hr.**

| OD (650 nm) Time [hr.] | OD_{I} | OD_{II} | OD_{III} | (OD_{III}-OD_{II}) (OD_{I}-OD_{II}) |
|---|---|---|---|---|
| 6 | 4.23 | 1.51 | 1.50 | -0.2% |
| 8 | 4.08 | 1.46 | 1.45 | -0.4% |

From the results it is clear that PEG 200 only very slowly or not at all metabolized by the strain used in this example.

**Table 3. Test of sucrose: Average OD (650 nm) found for the three shake flask at time 6 and 8 hr.**

| OD (650 nm) Time [hr.] | OD_{I} | OD_{II} | OD_{III} | (OD_{III}-OD_{II}) (OD_{I}-OD_{II}) |
|---|---|---|---|---|
| 6 | 4.23 | 1.51 | 4.23 | 100.1% |
| 8 | 4.08 | 1.46 | 4.02 | 97.7% |

From the results it is clear that sucrose is easy metabolized by the strain used in this example.

### Example 2

### Increased enzyme solubility in fermentation broth by addition of MPG to the fermentation process

### Strain: Bacillus licheniformis

Polypeptide of interest: an alpha-amylase variant described in WO 01/66712

### Media:

In all cases unless otherwise described tap water was used. All media were sterilized by methods known within the art to ensure that the fermentations were run as mono-cultures.

### First inoculum medium:

LB agar: 10 g/I peptone from casein; 5 g/I yeast extract; 10 g/I Sodium Chloride; 12 g/I Bacto-agar adjusted to pH 6.8 to 7.2. Premix from Merck was used.

### Transfer buffer:

M-9 buffer (deionized water is used): Di-Sodiumhydrogenphosphate, 2H2O 8.8 g/I; Potassiumdihydrogenphosphate 3 g/l; Sodium Chloride 4 g/l; Magnesium sulphate, 7H2O 0.2 g/l.

### Inoculum shake flask medium (concentration is before inoculation):

PRK-50: 110 g/I soy grits; Di-Sodiumhydrogenphosphate, 2H2O 5 g/I; pH adjusted to 8.0 with NaOH/H3PO4 before sterilization.

### Make-up medium (concentration is before inoculation):

Tryptone (Casein hydrolysate from Difco) 30 g/I; Magnesium sulphate, 7H2O 4 g/I; Di-Potassiumhydrogenphosphate 7 g/I; Di-Sodiumhydrogenphosphate, 2H2O 7 g/I; Di-Ammoniumsulphate 4 g/l; Citric acid 0.78 g/I; Vitamins (Thiamin-dichlorid 34.2 mg/l; Riboflavin 2.9 mg/l; Nicotinic acid 23 mg/l; Calcium D-pantothenate 28.5 mg/l; Pyridoxal-HCl 5.7 mg/l; D-biotin 1.1 mg/l; Folic acid 2.9 mg/l); Trace metals (MnSO4, H2O 39.2 mg/l; FeSO4, 7H2O 157 mg/l; CuSO4, 5H2O 15.6 mg/l; ZnCl2 15.6 mg/l); Antifoam (SB2121) 1.25 ml/l; pH adjusted to 6.0 with NaOH/H3PO4 before sterilization.

### Feed medium:

Glucose,1 H2O 820 g/I;

### Procedure for inoculum steps:

First the strain was grown on LB agar slants 1 day at 37°C.
The agar was then washed with M-9 buffer, and the optical density (OD) at 650 nm of the resulting cell suspension was measured.
The inoculum shake flask (PRK-50) is inoculated with an inoculum of OD (650 nm) x ml cell suspension = 0.1.
The shake flask was incubated at 37°C at 300 rpm for 20 hr.
The fermentation in the main fermentor (fermentation tank) was started by inoculating the main fermentor with the growing culture from the shake flask. The inoculated volume was 10% of the make-up medium (80 ml for 800 ml make-up media).

### Fermentor Equipment:

Standard lab fermentors were used equipped with a temperature control system, pH control with ammonia water and phosphoric acid, dissolved oxygen electrode to measure >20% oxygen saturation through the entire fermentation.
Fermentation parameters:
Temperature: 41°C
The pH was kept between 6.8 and 7.2 using ammonia water and phosphoric acid
Control: 6.8 (ammonia water); 7.2 phosphoric acid
Aeration: 1.5 liter/min/kg broth weight
Agitation: 1500 rpm
Feed strategy:
0 hr. 0.05 g/min/kg initial broth after inoculation
8 hr. 0.156 g/min/kg initial broth after inoculation
End 0.156 g/min/kg initial broth after inoculation
Experimental setup:
Three fermentations were run in parallel all with the same inoculation material.
Fermentation A was run as described above.
Fermentation B was run as described above but 50 g/L MPG (monopropyleneglycol) was added to the make-up medium before inoculation.
Fermentation C was run as described above but 50 g/L MPG was added to the fermentation 24 hours after inoculation.
(In fermentation Band C, the concentrations of MPG is given as the concentration based on the volume of the make-up medium before inoculation.)
Samples were taken after 3 days of fermentation. The samples were split in two identical parts (sample I and sample II). Sample II was centrifuged at 15000 gₐᵥ in 20 minutes at 38°C. The resulting supernatant was then filtered through a 0.2µm filter (Sartorius Minisart, order no.: 16534) (sample IIₛᵤₚ).
The alpha-amylase activities in sample I and sample IIₛᵤₚ was then measured by methods known within the art (for example method for alpha-amylase activity measurements described in WO 95/26397 can be used). However, when measuring samples where the enzyme can be in a partly solid form, samples have to be treated with urea prior to analysis. By diluting the samples 1:50 (w/v) in a solution containing 40% (w/w) urea, 25.5 mg/L Brij 35 and 4.4 g/L CaCl2, 2H2O both crystalline and precipitated alpha-amylase is brought into solution in an active form and it can then be further diluted in the buffer used in the activity assay.
Both sample I and sample II_{sub} are diluted in the urea-buffer as described above before being analysed.
The enzyme activity in sample IIₛᵤₚ is a measure of the soluble activity, whereas the activity in sample I is a measure of the total activity (both soluble and crystallized and precipitated activity).

### Results:

The following activities were found after 3 days of fermentation. The total activity found in fermentation A is used set to 100% and the soluble activity (i.e. the activity in sample IIₛᵤₚ) is given relative to the activity in sample I.

**Table 4.**

| Fermentation name | Description | Enzyme activity in total broth Sample I | Enzyme activity in solution Sample IIₛᵤₚ |
|---|---|---|---|
| Fermentation A | Standard | 100 % | 19 % |
| Fermentaion B | Addition of MPG to make-up media | 101 % | 53 % |
| Fermentation C | Addition of MPG after 1 day of fermentation | 99 % | 95 % |

From the results in Table 4 it is clear that addition of MPG to the media have a very significant effect on the amount of enzyme in solution in the fermentation broth.

### EXAMPLE 3

### Evaluation of the improved recovery Yield and Process

Two fermentations made in a scale larger than 50 liter were made with a *Bacillus* strain that produces an alpha-amylase with a low solubility. One of the fermentations had an addition of 2 % (w/w) MPG 24 hours after inoculation; here enzyme crystals were not visible in a microscope (40X). The other fermentation was a reference fermentation without any addition of MPG; here crystals were visible in the broth in a microscope (40X).
The two batches were treated equal in a series of flocculations where pH was adjusted to 4 different set points and all additions are in % w/w. The flocculation consisted of an addition of 200 % water; 2 % CaCl2; 1.2 % Al2(OH)5Cl; pH adjustment to a set point (see table below); 0.5 % Superfloc C591 and 0.2 to 0.3 % of Superfloc A130 depending of the pH. All flocculations were based on 100 g culture broth and all additions were done under stirring conditions. The flocculations were done at room temperature (approximately 20°C). The samples were centrifuged and the yield of the alpha-amylase activity were determined in the supernatant fractions.

**Table 5: Flocculation at different pH to illustrate the effect of MPG added to the fermentation on supernatant (centrate) yield.**

| **Flocculation pH** | **Supernatant yield MPG added to fermentation broth** | **Supernatant yield reference batch** |
|---|---|---|
| 7.5 | 83 % | 30 % |
| 10.0 | 102 % | 33 % |
| 10.5 | 100 % | 37 % |
| 11.0 | 98 % | 40 % |

In an industrial scale the sludge from the first centrifugation would be reflocculated and centrifuged again to increase the total separation yield. The sludge from the flocculation trials with pH 7.5 (mentioned above) was resuspended in 150 % water and 1 % CaCl2 and finally flocculated with 0.1 to 0.2 % Superfloc A130. Initially the sludge from the flocculation based on the broth without MPG added to the fermentation had 3x more activity than the sludge that came from flocculation based on the fermentation with MPG added to it. Again crystals were visible in a microscope (40X) in the sludge coming from the fermentation without MPG addition whereas the sludge from the batch coming from the fermentation with MPG did not contain any visible crystals in a microscope (40X).
Again the samples were centrifuged and the yield of the alpha-amylase activity in the reflocculated sludge was determined in the supernatant fractions.

**Table 6: Flocculation of sludge from first flocculation at different pH to illustrate the effect of MPG added to the fermentation.**

| **Flocculation pH** | **Supernatant yield MPG added fermentation broth** | **Supernatant yield reference batch** |
|---|---|---|
| 7.5 | 52 % | 6 % |
| 10.0 | 61 % | 7 % |
| 10.5 | 64 % | 10% |
| 11.0 | 67 % | 10 % |

As illustrated in this example the yield is much higher in the recovery of the enzyme if the crystal formation can be reduced or eliminated by addition of MPG during fermentation.

### EXAMPLE 4

### Effect of adding both MPG and a mineral salt to the fermentation broth

### Materials and methods:

A fermentation method similar to the one described in Example 2 (fermentation A) was used as the basis for these experiments.

**Table 7. Experimental setup:**

| Fermentation name | Addition of MPG after 1 day of fermentation ¹⁾ | Extra mineral added ²⁾ | Amount ³⁾ | |
|---|---|---|---|---|
| | | | [g/l] | [mol/l] |
| D | Yes | No | - | - |
| E | Yes | NaCl | 12 | 0.21 |
| F | Yes | KCI | 15 | 0.20 |
| G | Yes | Na₂SO₄ | 14 | 0.10 |
| H | Yes | K₂SO₄ | 18 | 0.10 |
| I | No | NaCl | 12 | 0.21 |

| | | | | |
|---|---|---|---|---|
| 1) This indicates if MPG is added to the fermentation 1 day after inoculation. The amount of MPG added is 50 g/I MPG based on the volume of the make-up media before inoculation. 2) The extra mineral or extra amount of an already added mineral added to main media before inoculation. 3) Salt concentration, amounts are based on volume of the make-up media before inoculation. | | | | |

### Experimental setup:

Fermentation D is the reference process for this study, while the fermentations E, F, G, H, I had extra mineral salt added to the make-up media in the amounts found in table 7. Samples were taken after 3 days of fermentation. The samples were split into two identical parts (sample I and sample II). Sample II was centrifuged at 15000 gₐᵥ in 20 minutes at 38°C. The resulting supernatant was then filtered through a 0.2µm filter (Sartorius Minisart, order no.: 16534) (sample IIₛᵤₚ).
The alpha-amylase activities in sample I and sample IIₛᵤₚ was then measured by methods known within the art (for example method for alpha-amylase activity measurements described in WO 95/26397 can be used). However, when measuring samples where the enzyme can be in a partly solid form, samples have to be treated with urea prior to analysis. By diluting the samples 1:50 (w/v) in a solution containing 40% (w/w) urea, 25.5 mg/L Brij 35 and 4.4 g/L CaCl2, 2H2O both crystalline and precipitated alpha-amylase is brought into solution in an active form and it can then be further diluted in the buffer used in the activity assay.
Both sample I and sample II_{sub} were diluted in the urea-buffer as described above before being analysed.
The enzyme activity in sample IIₛᵤₚ is a measure of the soluble activity, whereas the activity in sample I is a measure of the total activity (both soluble and crystallized and precipitated activity).

### Results:

**Table 8: The following activities were found after 3 days of fermentation. The total activity found in fermentation D is set to 100%, and the soluble activity (i.e. the activity in sample IIₛᵤₚ) is given relative to the activity in sample I for the specific fermentation.**

| Fermentation name | Description | Enzyme activity in total broth Sample I | Enzyme activity in solution Sample IIₛᵤₚ |
|---|---|---|---|
| Fermentation D | Addition of MPG after 1 day of fermentation (reference) | 100 | 39 |
| Fermentaion E | Type D + NaCl in makeup | 96 | 156 |
| Fermentation F | Type D + KCI in makeup Type | 88 | 149 |
| Fermentation G | Type D + Na2SO4 in makeup | 99 | 148 |
| Fermentation H | Type D + K2SO4 in makeup | 82 | 152 |
| Fermentation I | NaCl in makeup, no MPG | 109 | 15 |

The reason for the activity being higher in sample II, compared to sample I in some cases is that in the cases where all enzyme is in solution, removal of the cells increases the enzyme concentration in the sample, as the cells have a significant volume, but only a low enzyme concentration.
The data in table 8 clearly show that the increased enzyme solubility achieved by the MPG addition (D versus I : 39% versus 15%; and E versus I: 156 % versus 15 %). It is also clear that the increased solubility of the enzyme achieved by the MPG addition can be enhanced by the addition of various minerals.
Table 8 shows that there is a synergistic effect (see Fermentation D (MPG added); Fermentation I (NaCl added); and Fementation E (MPG and NaCl added)).

### EXAMPLE 5

A solution of alpha-amylase (e.g. recovered from the broth described in Example 2 by conventional means) is concentrated at pH 10.5-11 at 40°C. The concentrate is filtered through a 0.2µm filter at 40°C. Aliquots of 25 mL is transferred to 7 vials, to which is then added one of the following polyols:

| **Vial no.** | **Polyol** |
|---|---|
| 1 | reference |
| 2 | 5% (w/w) PEG 200 |
| 3 | 5% (w/w) PEG 400 |
| 4 | 5% (w/w) sorbitol |
| 5 | 5% (w/w) cellobiose |
| 6 | 5% (w/w) xylitol |
| 7 | 5% (w/w) monopropylene glycol (MPG) |

A magnetic bar is placed in each vial, and the pH is adjusted to pH 7.5. The vials are then left at room temperature with slow stirring for two days.

The crystals are then removed by filtration (0.2 µm filters) and the activity is measured both in the filtrate (= mother liquor) and in the sample before filtration. The results presented in the table below are given as the activity in the mother liquor relative to the total activity in the sample before filtration (in %).

**Table 9.**

| Vial no. | Polyol added | Activity in mother liquor relative to total activity (%) |
|---|---|---|
| 1 | Reference | 9 |
| 2 | 5% (w/w) PEG 200 | 58 |
| 3 | 5% (w/w) PEG 400 | 53 |
| 4 | 5% (w/w) sorbitol | 33 |
| 5 | 5% (w/w) cellobiose | 38 |
| 6 | 5% (w/w) xylitol | 29 |
| 7 | 5% (w/w) monopropylene glycol (MPG) | 29 |

The data clearly demonstrate that addition of polyols increases the solubility of the amylase.

## Claims

1. A method for fermenting a bacterium, producing an enzyme of interest, in a culture medium of at least 50 litres, comprising:
adding one or more compounds selected from the group consisting of 1,2-propandiol, 1,3-propandiol, ethylene glycol, trehalose, xylitol, arabitol, dulcitol, mannitol, erythritol, cellobiose, sorbitol and a polyether having an average molecular weight less than 1000, to the culture medium during fermentation, wherein the compound is low metabolizable measured by (OD_{III}-OD_{II})/(OD_{I}-OD_{II}) < 25%, and added in an amount of 0.5-10 % (w/w) of the culture medium, wherein
OD_{I} is the amount of biomass measured as optical density (OD) at 650 nm with glucose as the only carbohydrate source;
OD_{II} is the amount of biomass measured as optical density (OD) at 650 nm without glucose; and
OD_{III} is the amount of biomass measured as optical density (OD) at 650 nm without glucose, but with the same C-mole of the compound to be tested".

2. The method according to claim 1, wherein the bacterium is a *Bacillus* strain.

3. The method according to claim 1, wherein the enzyme is a hydrolase (class EC 3 according to Enzyme Nomenclature).

4. The method according to claim 1, wherein the compound is 1,2-propandiol.

5. The method according to claim 1, wherein in addition to the compound a salt is added to the fermentation medium.

6. The method according to claim 5, wherein the salt is selected from the group consisting of a chloride, a sulphate, a phosphate, a nitrate, and an ammonium salt.

7. The method according to claim 1, wherein the enzyme of interest is recovered.

8. The method according to claim 1, wherein the enzyme is recovered after removal of the bacterium.

## Patentansprüche

1. Verfahren zum Fermentieren eines Bakteriums, das ein Enzym von Interesse herstellt in einem Kulturmedium von mindestens 50 1, wobei das Verfahren die folgenden Schritte umfasst:
Hinzufügen einer oder mehrerer Verbindungen zu dem Kulturmedium während der Fermentation, ausgewählt aus der Gruppe bestehend aus 1,2-Propandiol, 1,3-Propandiol, Ethylenglykol, Trihalose, Xylitol, Arabitol, Dulcitol, Mannitol, Erythrit, Cellobiose, Sorbit und einem Polyether mit einem mittleren Molekulargewicht von weniger als 1000, wobei die Verbindung gering metabolisierbar ist, gemessen durch (OD_{III}-OD_{II})/(OD_{I}-OD_{II}) < 25 % und die in einer Menge von 0,5-10 Gew.-% des Kulturmediums hinzugefügt wird/werden, wobei
OD_{I} die Menge an Biomasse ist, die als optische Dichte (OD) bei 650 nm mit Glucose als der einzigen Kohlenhydratquelle gemessen wird;
OD_{II} die Menge an Biomasse ist, die als optische Dichte (OD) bei 650 nm ohne Glucose gemessen wird; und
OD_{III} die Menge an Biomasse ist, die als optische Dichte (OD) bei 650 nm ohne Glucose, aber mit dem gleichen C-Mol der zu testenden Verbindung gemessen wird.

2. Verfahren nach Anspruch 1, wobei das Bakterium ein Bacillus-Stamm ist.

3. Verfahren nach Anspruch 1, wobei das Enzym eine Hydrolase (Klasse EC 3 entsprechend der Enzymnomenklatur) ist.

4. Verfahren nach Anspruch 1, wobei die Verbindung 1,2-Propandiol ist.

5. Verfahren nach Anspruch 1, wobei zusätzlich zu der Verbindung ein Salz zu dem Fermentationsmedium hinzugefügt wird.

6. Verfahren nach Anspruch 5, wobei das Salz ausgewählt ist aus der Gruppe bestehend aus einem Chlorid-, einem Sulfat-, einem Phosphat-, einem Nitrat- und einem Ammoniumsalz.

7. Verfahren nach Anspruch 1, wobei das Enzym von Interesse rückgewonnen wird.

8. Verfahren nach Anspruch 1, wobei das Enzym nach Entfernung des Bakteriums rückgewonnen wird.

## Revendications

1. Procédé pour fermenter une bactérie, produisant un enzyme d'intérêt, dans un milieu de culture d'au moins 50 litres, comprenant :
L'ajout d'un ou plusieurs composés sélectionnés parmi le groupe constitué de 1,2-propanediol, 1,3-propanediol, éthylène glycol, tréhalose, xylitol, arabitol, dulcitol, mannitol, érythritol, cellobiose, sorbitol et un polyéther ayant un poids moléculaire moyen inférieur à 1000, au milieu de culture pendant la fermentation, le composé étant faiblement métabolisable ce qui est mesuré par (OD_{III}-OD_{II})/(OD_{I}-OD_{II}) < 25%, et ajouté en une quantité de 0,5-10 % (p/p) au milieu de culture, dans lequel,
OD_{I} est la quantité de biomasse mesurée en densité optique (OD) à 650 nm avec le glucose comme seule source de carbohydrate ;
OD_{II} est la quantité de biomasse mesurée en densité optique (OD) à 650 nm sans glucose ;
OD_{III} est la quantité de biomasse mesurée en densité optique (OD) à 650 nm sans glucose, mais avec la même C-mole du composé à tester.

2. Procédé selon la revendication 1, dans lequel la bactérie est un une souche *Bacillus.*

3. Procédé selon la revendication 1, dans lequel l'enzyme est une hydrolase (classe EC 3 selon la Nomenclature des Enzymes).

4. Procédé selon la revendication 1, dans lequel le composé est le 1,2-propanediol.

5. Procédé selon la revendication 1, dans lequel en plus du composé, un sel est ajouté au milieu de fermentation.

6. Procédé selon la revendication 5, dans lequel le sel est sélectionné parmi le groupe constitué d'un chlore, un sulfate, un phosphate, un nitrate, et un sel d'ammonium.

7. Procédé selon la revendication 1, dans lequel l'enzyme d'intérêt est récupéré.

8. Procédé selon la revendication 1, dans lequel l'enzyme est récupéré après élimination de la bactérie.
